(19) Europäisches Patentamt / European Patent Office / Office européen des brevets

(11) **EP 3 988 933 B1**

(12) **EUROPEAN PATENT SPECIFICATION**

(45) Date of publication and mention of the grant of the patent: **16.09.2026 Bulletin 2026/38**

(21) Application number: **20827280.7**

(22) Date of filing: **19.06.2020**

(51) International Patent Classification (IPC):
*G01N 33/48* (2006.01) *G01N 33/86* (2006.01)
*G06N 20/00* (2019.01) *G16H 10/60* (2018.01)
*G16H 50/20* (2018.01) *G16H 10/40* (2018.01)

(52) Cooperative Patent Classification (CPC):
**G01N 33/86; G06N 20/00; G16H 10/40; G16H 10/60; G16H 50/20**

(86) International application number:
**PCT/JP2020/024148**

(87) International publication number:
**WO 2020/256107 (24.12.2020 Gazette 2020/52)**

(54) **APTT PROLONGATION FACTOR ESTIMATION SYSTEM**

SYSTEM ZUR APTT-VERLÄNGERUNGSFAKTORSCHÄTZUNG

SYSTÈME D’ESTIMATION DE FACTEUR DE PROLONGATION D’APTT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.06.2019 JP 2019114425**

(43) Date of publication of application:
**27.04.2022 Bulletin 2022/17**

(73) Proprietors:
- **Sekisui Medical Co., Ltd.**
  **Tokyo 103-0027 (JP)**
- **Public University Corporation Nara Medical University**
  **Nara 634-8521 (JP)**

(72) Inventors:
- **KAWABE, Toshiki**
  **Tokyo 103-0027 (JP)**
- **ODA, Yukio**
  **Tokyo 103-0027 (JP)**
- **SHIMA, Midori**
  **Kashihara-shi, Nara 634-8521 (JP)**
- **NOGAMI, Keiji**
  **Kashihara-shi, Nara 634-8521 (JP)**
- **OGIWARA, Kenichi**
  **Kashihara-shi, Nara 634-8521 (JP)**
- **SHIMONISHI, Naruto**
  **Kashihara-shi, Nara 634-8521 (JP)**

(74) Representative: **Wächtershäuser & Hartz Patentanwaltspartnerschaft mbB**
**Weinstraße 8**
**80333 München (DE)**

(56) References cited:
**JP-A- 2002 149 833** **JP-A- 2002 259 573**
**JP-A- 2013 250 903** **JP-A- 2013 250 904**
**JP-A- 2017 021 744** **JP-A- 2019 086 518**
**JP-A- H1 145 304** **US-A1- 2010 228 138**
**US-A1- 2013 344 519** **US-A1- 2016 274 133**
**US-B1- 6 524 861** **US-B1- 6 898 532**

- **LASSON FRANCOIS ET AL: "A Clinical Decision Support System to Help the Interpretation of Laboratory Results and to Elaborate a Clinical Diagnosis in Blood Coagulation Domain", 16 May 2019, ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 109 - 122, ISBN: 978-3-319-10403-4, XP047509600**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HAGIWARA, KENICHI ET AL.: "Development of Hemophilia A Diagnostic Algorithm Using APTT Clot Waveform Template Matching", NIHON KESSEN SHIKETSU GAKKAISHI - JAPANESE JOURNAL OF THROMBOSIS AND HEMOSTASIS, vol. 30, no. no. 2, 1 May 2019 (2019-05-01), JP , pages 411, XP009532285, ISSN: 0915-7441
- SHIMONISHI NARUTA : "O-096 Rapid Quantification of APTT Cross-Mixing Tests Using Clot Waveform Analysis Baycentric Method Parameters", JAPANESE JOURNAL OF THROMBOSIS AND HEMOSTASIS, vol. 30, no. 2, 1 May 2019 (2019-05-01), pages 440, XP009527868, ISSN: 0915-7441

## Description

Field of the Invention

The present invention relates to an APTT prolongation factor estimation system.

Background of the Invention

A blood coagulation test is a test for diagnosing a blood coagulation function of a patient by adding a predetermined reagent to a blood specimen of the patient and measuring a blood coagulation time or the like. Through the blood coagulation test, the state of hemostatic ability and fibrinolytic ability of the patient can be grasped. As typical examples of the blood coagulation time, there are given a prothrombin time (PT), an activated partial thromboplastin time (APTT), a thrombin time, and the like. In recent years, an automatic analyzer configured to perform automatic measurement in the blood coagulation test has also been widely used, and hence the blood coagulation test can be easily performed.

As causes for prolongation of the blood coagulation time, there are given the effect of an anticoagulant, the reduction in a component involved in coagulation, the congenital deficiency of a blood coagulation factor, and the emergence of an acquired autoantibody that inhibits a coagulation reaction. For example, when APTT prolongation is found, a cross-mixing test is generally further performed to determine which of a coagulation factor inhibitor (anticoagulant factor), a lupus anticoagulant (LA), or coagulation factor deficiency, for example, hemophilia, has led to the APTT prolongation. In the cross-mixing test, normal plasma, test plasma, and mixed plasma containing the test plasma and the normal plasma in various volume ratios are measured for APTT immediately after mixing (immediate reaction) and APTT after incubation at 37°C for 2 hours (delayed reaction). The measurement values of the cross-mixing test are graphed with the vertical axis representing an APTT measurement value (seconds) and the horizontal axis representing a volume ratio of the test plasma to the normal plasma. The created graphs of the immediate reaction and the delayed reaction each show a "downward convex", "straight line", or "upward convex" pattern depending on the APTT prolongation factor. The APTT prolongation factor is determined based on the immediate reaction and delayed reaction patterns.

When it is determined that the APTT prolongation is caused by a coagulation factor inhibitor, an inhibitor titer is generally measured by a Bethesda method. In the Bethesda method, a sample obtained by mixing a diluted series of test plasma and normal plasma is incubated at 37°C for 2 hours. After that, the residual activity of a coagulation factor in the sample is measured, and an inhibitor titer of the coagulation factor is measured based on a calibration curve from the measurement value. Currently, the Bethesda method is a standard quantification method for an inhibitor titer regarding a coagulation factor VIII (FVIII) and a coagulation factor IX (FIX).

In the blood coagulation test, a coagulation reaction curve can be obtained by measuring a blood coagulation reaction amount after addition of a reagent to a blood specimen over time. The coagulation reaction curve has a different shape depending on the type of abnormality in a blood coagulation system (Non Patent Literature 1). Accordingly, there is disclosed a method of determining abnormality in the blood coagulation system based on the coagulation reaction curve. For example, in each of Patent Literatures 1 to 3 and Non Patent Literatures 2 to 4, there is described a method of evaluating the presence or absence of abnormality of a coagulation factor in a patient based on parameters related to a primary differential curve and a secondary differential curve of a coagulation reaction curve regarding the blood of the patient, such as a maximum coagulation rate, a maximum coagulation acceleration rate, a maximum coagulation deceleration rate, and arrival times thereof. In Patent Literature 4, there is described a method of determining the severity of hemophilia based on the average change rate of a coagulation rate until the time when the coagulation reaction of a patient reaches a maximum coagulation rate or a maximum coagulation acceleration rate. In Patent Literature 5, there is described a method of determining the presence of a FVIII inhibitor based on the ratio between the slope of a straight line representing the coagulation time with respect to the dilution factor of patient plasma and the slope of a straight line representing the coagulation time with respect to the dilution factor of control plasma. In Patent Literature 6, there are described that a plurality of parameters are extracted from a primary differential waveform and a secondary differential waveform of a blood coagulation waveform, and a predicted concentration of each coagulation factor is obtained from the plurality of parameters by multivariate backward correlation, and that a trained neural network is used for the prediction.

There is provided a method of centrally managing data from a plurality of analyzers and maintenance of those devices. In Patent Literature 7, there is described a method for managing accuracy of an analyzer, which involves receiving an accuracy management measurement value from a target analyzer and comparing the received measurement value to a statistical reference generated based on accuracy management measurement values from a plurality of analyzers, to thereby evaluate whether the measurement value of the target analyzer is normal or abnormal. In Patent Literature 8, there is described a method of evaluating a biological sample, which involves transmitting raw data on a biological sample containing a numerical value representing physical treatment or a chemical reaction to a cell performed by an instrument and an image of the cell from the instrument to an analytical facility, to thereby analyze the raw data in the analytical facility,

and monitoring the completeness of the analysis and the operation of the instrument.

US 6898532 B1 discloses a method for predicting the presence of haemostatic dysfunction utilising a trained model.

[PTL 1] JP-A-2016-194426
[PTL 2] JP-A-2016-118442
[PTL 3] JP-A-2017-106925
[PTL 4] JP-A-2018-017619
[PTL 5] JP-A-2018-517150
[PTL 6] US 6524861 B1
[PTL 7] JP-A-2017-187473
[PTL 8] JP-A-2016-508610

Non Patent Literature

[NPL 1] British Journal of Haematology, 1997, 98:68-73
[NPL 2] The Japanese Journal of Clinical Hematology, 2017, vol. 58, no. 9, p. 1754, PS2-37-7
[NPL 3] Japanese Journal of Thrombosis and Hemostasis, 2018, vol. 29, no. 2, p. 184, O-056, P-080
[NPL 4] Japanese Journal of Thrombosis and Hemostasis, 2018, vol. 29, no. 4, pp. 413-420

Summary of the Invention

Technical Problem

The present invention is to provide an APTT prolongation factor estimation system.

Solution to Problem

The inventors of the present invention provide the following Items.

An APTT prolongation factor estimation system as defined in appended claim 1.

An APTT prolongation factor estimation method as defined in appended claim 16.

Preferable embodiments of the invention are set out in the dependent claims.

Advantageous Effects of the Invention

According to the present invention, highly accurate APTT prolongation factor estimation based on a large number of findings is achieved based on a database of coagulation reaction curves from various samples. According to the present invention, the APTT prolongation factor can be precisely determined even in a medical institution having less experience in diagnosing APTT abnormality. Further, according to the present invention, the APTT prolongation factor can be determined only based on data of coagulation reaction curves even in a medical institution, a clinic, a facility, and the like in which a cross-mixing test and inhibitor titer measurement are not performed.

Brief Description of Drawings

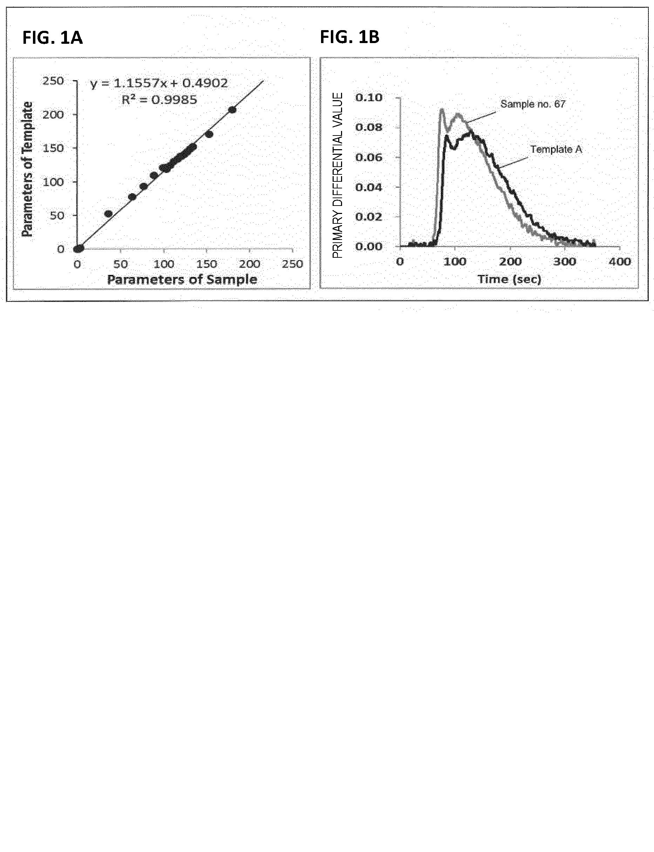

FIG. 1A is a graph for showing a regression line of a parameter group. FIG. 1B is a graph for showing primary differential curves of a subject specimen and a template. Sample No. 67 corresponds to the subject specimen, and Template A corresponds to the template.

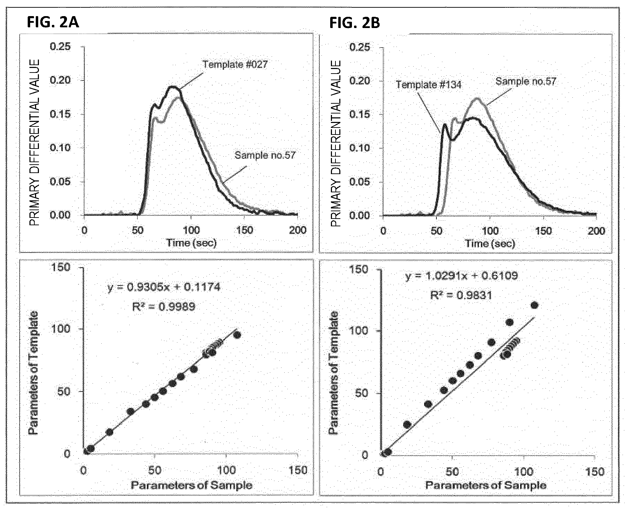

FIG. 2A is a graph for showing primary differential curves of a subject specimen (Sample No. 57) and a template (#027) (upper side) and a regression line of a parameter group (lower side). FIG. 2B is a graph for showing primary differential curves of the subject specimen (Sample No. 57) and a template (#134) (upper side) and a regression line of a parameter group (lower side).

Detailed Description of the Invention

1. APTT Prolongation Factor Estimation System

As an APTT prolongation factor, a coagulation factor inhibitor (anticoagulant factor), a lupus anticoagulant (LA), coagulation factor deficiency, for example, hemophilia, and the like can be usually estimated. In the determination of the APTT prolongation factor, the APTT prolongation factor has hitherto been generally determined based on APTT results obtained from a coagulation reaction curve by a cross-mixing test, inhibitor titer measurement by a Bethesda method, or the like. However, it is not easy to perform all the tests for determination within one medical institution because cost and manpower are required. Further, it is required to accumulate a large number of findings in order to secure and improve the accuracy of determination. However, the frequency of occurrence of patients having APTT abnormality, for example, patients having coagulation factor deficiency is very low, and hence it is difficult for only one medical institution to collect sufficient findings. Those problems may lead to variations in test results depending on medical institutions.

As described in Patent Literatures 1 to 5, there is disclosed a method of determining coagulation factor abnormality and the presence of a coagulation factor inhibitor based on parameters related to a waveform, such as a peak height, a time, or a slope, regarding a primary differential curve or a secondary differential curve of a coagulation reaction curve. Further, the applicants of the present application provided a method of performing determination of the APTT prolongation factor, inhibitor titer measurement, determination of the presence or absence of deficiency of a coagulation factor inhibitor, or determination of the type of a deficient inhibitor through use of parameters related to a waveform or a weighted average point (for example, a coagulation time, a weighted average time, a weighted average height, a peak width, a flatness, and a time rate, and a ratio or a difference thereof) regarding a coagulation reaction curve, or a primary differential curve thereof or a secondary differential curve thereof (for example, Japanese Patent Application No. 2019-086658, Japanese Patent Application No. 2019-084727, and Japanese Patent Application No. 2019-016474). Those methods not only enable determination of the APTT prolongation factor by an objective indicator based on various parameters calculated from a coagulation reaction curve measured by an analyzer, but also enable automation of the determination.

In order to determine the APTT prolongation factor based on the parameters related to a waveform or a weighted average point regarding a coagulation reaction curve, or a primary differential curve thereof or a secondary differential curve thereof more accurately, it is required to accumulate pieces of data of coagulation reaction curves from a large number of samples containing samples having normal and abnormal APTT and categorize the normal and abnormal waveforms and parameters. However, as described above, the frequency of occurrence of patients having APTT abnormality is low, and hence it is difficult for only one medical institution to collect sufficient pieces of data.

When the pieces of data from patients scattered in various places are collected to create a database, and the information thereof is shared, the determination accuracy can be improved because of a dramatical increase in number of pieces of accumulated data, and variations in test results depending on medical institutions can be reduced because of unified determination criteria.

1.1 Overview

The present invention is to provide an APTT prolongation factor estimation system. The APTT prolongation factor estimation system of the present invention comprises: one or more facilities each comprising an analyzer configured to measure a blood coagulation reaction of a blood specimen; a database configured to store data on a blood coagulation reaction of a blood specimen and APTT prolongation factor data; and a computer configured to estimate an APTT prolongation factor of the blood specimen based on data on the blood coagulation reaction from the analyzer and the data stored in the database. The facilities each further comprise: a data transmission unit configured to transmit, to the computer, the data on the blood coagulation reaction of the blood specimen obtained by the analyzer; and a data reception unit configured to receive a result of the estimation of the APTT prolongation factor, which is obtained by the computer, regarding the blood specimen measured by each of the analyzers.

The APTT prolongation factor estimation system of the present invention may further comprise: an analyzer control unit configured to control the analyzer; and/or a data display unit configured to display data received by the data reception unit.

The APTT prolongation factor estimation system of the present invention may further comprise an algorithm learning unit configured to machine-learn an APTT prolongation factor estimation algorithm through use of the data on the blood coagulation reaction of the blood specimen and the APTT prolongation factor data which are stored in the database.

A conceptual diagram of an APTT prolongation factor estimation system according to one embodiment of the present invention and an APTT prolongation factor estimation method using the system according to the one embodiment are shown in the following schematic diagram 1 and Table 1. In the schematic diagram 1, an analyzer configured to measure a blood coagulation reaction of a blood specimen and a communication device comprising a data transmission unit and a data reception unit are installed in each of three facilities A, B, and C. Each of the facilities is configured to transmit data on the blood coagulation reaction measured by the analyzer to a server common to the three facilities through

the data transmission unit. In the schematic diagram 1, the server is connected to the three facilities, but the number of the facilities connected to the server may be increased or decreased in accordance with the processing ability of the server, and is not particularly limited. The data on the blood coagulation reaction acquired by the server is transmitted to a computer, and the computer is configured to perform APTT prolongation factor estimation through use of the data and data stored in a database (DB). Each of the computer and the database may be incorporated into the server, or may be installed separately from the server. In the schematic diagram 1, the computer and the database are incorporated into the server. A result of the estimation of the APTT prolongation factor obtained by the computer may be transmitted from the server and received by the data reception unit of the facility. In addition, the result of the estimation of the APTT prolongation factor is accumulated in the database. The facility can determine an APTT prolongation factor of a blood specimen based on the received result of the estimation of the APTT prolongation factor. Results of the determination obtained in the facility can be accumulated in the database through the data transmission unit and the server. The server may further comprise an algorithm learning unit, or may be connected to an external algorithm learning unit. The algorithm learning unit may construct an APTT prolongation factor estimation algorithm by machine learning using the data stored in the database and provide the APTT prolongation factor estimation algorithm to the computer.

Schematic Diagram 1

Network
Facility A
Facility B
Facility C
Analyzer
Analyzer
Analyzer
Communi cation device
Communi cation device
Communi cation device
Server
⑤
①
②
④
⑥
③

Table 1

| Step | Treatment posi tion/Treatment method | Details of treatment |
|---|---|---|
| (1) | Analyzer | APTT measurement of subject specimen |

(continued)

| Step | Treatment posi tion/Treatment method | Details of treatment |
|---|---|---|
| (2) | Communication device → serve r | Data to be transmitted is data on the subject specimen having prolonged APTT (coagulation time) or data on a controlled specimen. The measurement of the controlled specimen is preferably measurement before that of the subject specimen in terms of time, which is measurement immediately before that of the subject specimen in terms of time. Here, the prolongation refers to the case in which the APTT exceeds a reference standard range. For example, APTT is longer than 39 seconds.<br>Data to be transmitted to server<br>a: Analysis condition<br>i) Identification number and photometric module number of analyzer<br>ii) Identification number and lot number of reagent<br>b: Specimen information<br>iii) Subject specimen or controlled specimen<br>c: APTT measurement data (measurement data) |
| (3) | Role of server (computer, da tabase) | 1) Configuration<br>·DB data<br>·Classification information ("waveform classification identification mark and index value thereof" obtained by analyzing DB data)<br>Example) Waveform classification identification mark "F08": FVIII concentration<br>Index value "01": <1%, "02": <1-5%, "03": <5-40%, "E": other<br>2) Data storage (database: DB)<br>·"APTT prolongation factor" and "APTT measurement data" are stored so as to be associated with each other. The stored data is referred to as "DB data".<br>3) Calculation of parameters related to waveform and weighted average point (when calculation is not performed in Step (1))<br>·A velocity waveform is calculated from measurement data, and parameters related to a waveform and a weighted average point are calculated based on the velocity waveform.<br>4) Search<br>Under "search conditions" set in advance, parameters of the data on the subject specimen received in Step (2) are checked against the DB data so as to find which DB data is matched with (approximate to) the parameters, and the matched DB data is selected.<br>·"Estimation results" are selected by an approximate index value based on the selected DB data.<br>5) Data accumulation<br>·When "APTT prolongation factor" information on the subject specimen determined in a facility is received in Step (6), the subject specimen data is accumulated as DB data. |
| (4) | Server → commu nication devic e | There are the following cases depending on the search condition results.<br>i) When there is matched DB data: there is one factor name, and a factor classification index value, and an approximate index value are listed.<br>ii) When there is matched DB data: there are a plurality of factor names, and factor classification index values and approximate index values are listed.<br>iii) When there is no matched DB data: "impossible to estimate" |
| (5) | Doctor | A doctor determines an APTT prolongation factor. |
| (6) | Communication device → serve r | A factor classification name, a factor cla ssification index value, and a factor name are transmitted based on the determined A PTT prolongation factor. |

The facilities encompass any facility that is required to perform blood coagulation reaction measurement or acquire and analyze measurement results, for example, medical institutions such as hospitals and clinics, testing facilities, research facilities, and the like.

As the analyzer, a commercially available blood analyzer capable of measuring a coagulation reaction of a blood specimen (for example, a plasma sample) is used. The analyzer can preferably perform the following measurements:

1) Measurement of coagulation reaction over time based on photometric data or the like

A coagulation reaction curve may also be calculated from measurement data.

2) Activated partial thromboplastin time (APTT) measurement

Depending on the analyzer, when the APTT measurement results of the specimen are prolonged, the next immediate reaction and delayed reaction can be automatically performed.

i) APTT measurement of subject specimen
ii) APTT measurement when mixed plasma of normal plasma and subject specimen is not subjected to heating treatment in order to check presence or absence of immediate reaction
iii) APTT measurement after mixed plasma of normal plasma and subject specimen is subjected to heating treatment in order to check presence or absence of delayed reaction

As a blood analyzer capable of performing the above-mentioned measurements 1) and 2), there is given, for example, a blood coagulation automatic analyzer CP3000 (Sekisui Medical Co., Ltd.) or the like.

As the data to be transmitted to the computer, there is given coagulation reaction measurement data (measurement data) over time on the blood specimen. The measurement data may be measured raw data, but may also be data of a coagulation reaction curve calculated from the measured raw data or a primary differential curve thereof or a secondary differential curve thereof, parameters related to a waveform or a weighted average point regarding the coagulation reaction curve or the primary differential curve thereof or the secondary differential curve thereof, or a combination thereof.

As the parameters related to a waveform or a weighted average point, there are given parameters related to a waveform (a coagulation time calculated from the coagulation reaction curve, a maximum peak height and time thereof regarding the primary differential curve or the secondary differential curve, and the like) and parameters related to a weighted average point (a weighted average time, a weighted average height, a peak width, a flatness, a time rate, and the like regarding the primary differential curve or the secondary differential curve). The parameters related to a waveform or a weighted average point are described later in the section "1.2.2".

When a cross-mixing test is performed, a normal specimen, a subject specimen, and a mixed specimen containing the subject specimen and the normal specimen in various volume ratios are measured for a coagulation reaction before heating treatment (immediate reaction) and a coagulation reaction after the heating treatment (delayed reaction), and the results may be transmitted as measurement data. Further, the above-mentioned parameters related to a waveform or a weighted average point before and after the heating treatment may be transmitted as measurement data, or a difference or a ratio between the obtained parameters before and after the heating treatment may be calculated and transmitted as measurement data.

The mixed specimen to be used in the cross-mixing test contains a subject specimen and a normal specimen in a volume ratio of from 1:9 to 9:1, preferably from 4:6 to 6:4, more preferably 5:5. As required, the subject specimen may be diluted before being mixed with the normal specimen, and the diluted subject specimen and the normal specimen may be mixed in the above-mentioned ratios. The heating treatment is performed, for example, at 30°C or more and 40°C or less, preferably 35°C or more and 39°C or less, more preferably 37°C within 1 hour, preferably a range of from 2 minutes to 30 minutes, more preferably from 5 minutes to 30 minutes. However, prior to the above-mentioned heating treatment, the specimen may be subjected to heating treatment in normal coagulation reaction measurement, for example, heating at 30°C or more and 40°C or less for 1 minute or less.

In addition to the measurement data described above, measurement conditions (for example, an analyzer identification number, a photometric module number, and the type, identification number, and lot number of a reagent used), data on whether or not the APTT of a blood specimen has been delayed, and the like may be transmitted as required.

It is only required that the blood specimen be a blood specimen derived from a test subject (hereinafter sometimes referred to as "subject specimen") that is required to be estimated for an APTT prolongation factor. Preferably, the subject specimen is a blood specimen indicating APTT prolongation. As the blood specimen, citrated plasma is preferred. Measurement data on the controlled specimen may be transmitted for calibration of the measurement results. As the controlled specimen, a blood specimen in which the presence or absence of APTT prolongation and a prolongation factor are known is used. Through comparison of the measurement data from the controlled specimen to the measurement

results from the past same specimen (or a specimen having the same prolongation factor), the measurement data of the analyzer of the facility can be calibrated and whether or not there is any abnormality in the analysis conditions can be inspected. The transmission of the controlled specimen data may be performed for each transmission of the subject specimen data or may be performed separately from the transmission of the subject specimen data, for example, periodically or in accordance with the reagent exchange. In the former case, the measurement of the controlled specimen is preferably performed before the subject specimen, more preferably immediately before the subject specimen.

The above-mentioned parameters related to a waveform or a weighted average point may be calculated in each facility and transmitted to the computer, or may be calculated by the computer through use of the raw data of the coagulation reaction measurement transmitted from each facility, the data of the coagulation reaction curve, or the primary differential curve thereof or the secondary differential curve thereof. In the following description, the raw data of the coagulation reaction measurement, the data of the coagulation reaction curve, or the primary differential curve or the secondary differential curve thereof, and the data on the parameters related to a waveform or a weighted average point of each of those curves are collectively referred to as "measurement data" without distinguishing between those derived from the facility and those calculated by the computer.

The estimation of the APTT prolongation factor by the computer may be performed based on the measurement data on the subject specimen having the coagulation reaction measured in each facility and the data stored in the database.

The database includes data such as the state of a specimen acquired in the past (for example, normality, the presence or absence of various coagulation factor inhibitors, an inhibitor titer, a LA, coagulation factor deficiency, the type of a deficient coagulation factor, the concentrations of various coagulation factors, and the like), and a coagulation reaction curve regarding the specimen or a primary differential curve thereof or a secondary differential curve thereof, or parameters related to a waveform or a weighted average point calculated from those curves.

For example, through comparison of the measurement data on the subject specimen to the measurement data on various specimens comprising normal specimens and specimens having various APTT prolongation factors stored in the database, an APTT prolongation factor of the subject specimen can be estimated. For example, the shape of the coagulation reaction curve, or the primary differential curve thereof or the secondary differential curve thereof may be directly compared between the subject specimen and the specimen in the database, or the parameters related to a waveform or a weighted average point thereof may be compared therebetween. From the results of the comparison, the APTT prolongation factor of the subject specimen can be estimated. For example, a specimen having a high parameter matching rate with the subject specimen is selected from the database, and the APTT prolongation factor (for example, coagulation factor deficiency, a LA, or a coagulation factor inhibitor) of the selected specimen may be estimated as the APTT prolongation factor of the subject specimen. The detailed procedure for estimating the APTT prolongation factor by the computer is described later.

As typical examples of APTT prolongation factors to be estimated, there are given coagulation factor deficiency, a LA, and possession of coagulation factor inhibitors. Further, more detailed factors as shown in the following Table 2 can also be estimated.

Table 2

| APTT prolongation factor to be estimated | | | |
|---|---|---|---|
| | Factor classification name | Factor classification index value | Factor name |
| 1 | FVIII Deficiency | FVIII:C <1 % | Severe hemophilia A |
| 2 | FVIII Deficiency | FVIII:C 1% or more and less than 5% | Moderate hemophilia A |
| 3 | FVIII Deficiency | FVIII:C 5% or more and less than 40% | Mild hemophilia A |
| 4 | FIX Deficiency | FIX:C <1 % | Severe hemophilia B |
| 5 | FIX Deficiency | FIX:C 1% or more and less than 5% | Moderate hemophilia B |
| 6 | FIX Deficiency | FIX:C 5% or more and less than 40% | Mild hemophilia B |
| 7 | Deficiency of other factors (F**) | F**:C #-# % | F** deficiency |
| 8 | LA | Dilute Russell's viper venom time (dRVVT (ratio)) | LA |
| 9 | FVIII Inhibitor | Titer ≤5 (BU/mL) | FVIII Inhibitor H |
| 10 | FVIII Inhibitor | Titer >5 (BU/mL) | FVIII Inhibitor L |
| 11 | FIX Inhibitor | Titer ≤5 (BU/mL) | FIX Inhibitor H |

(continued)

| APTT prolongation factor to be estimated | | | |
|---|---|---|---|
| | Factor classification name | Factor classification index value | Factor name |
| 12 | FIX Inhibitor | Titer >5 (BU/mL) | FIX Inhibitor L |
| **: Factors (II, V, X, XI, XII, prekallikrein) other than VIII and IX<br>#: Factor activity value<br>FVIII:C, FIX:C, and F**C each mean a coagulation factor concentration. | | | |

The results of the estimation of the APTT prolongation factor regarding the subject specimen calculated by the computer are transmitted to the facility that has acquired the data on the subject specimen. In addition to the factor name or factor classification name shown in Table 2, the results of the estimation may comprise a factor classification index value, a maximum matching rate of the parameters with the specimen in the database at the time when the results the estimation are obtained, and statistical indices (a correlation coefficient, a coefficient of determination, a significant difference, and the like). Even when the prolongation factor is not determined to be a single factor, a plurality of prolongation factors are estimated, or a prolongation factor cannot be estimated, the information to that effect may be transmitted to the facility while including information such as the maximum matching rate of the parameters and statistical indices (a correlation coefficient, a coefficient of determination, a significant difference, and the like) as required.

The results of the estimation of the APTT prolongation factor transmitted from the computer are received by the data reception unit of the facility, and are stored or used. For example, based on the results of the estimation, a doctor in the facility can determine the APTT prolongation factor of the subject specimen.

The results of the estimation of the APTT prolongation factor regarding the subject specimen calculated by the computer may be accumulated in the database. The database may be updated so as to newly add the measurement data (data of a coagulation reaction curve or a primary differential curve thereof or a secondary differential curve thereof, or parameters related to a waveform or a weighted average point calculated from those curves, and the like) regarding the subject specimen transmitted from the computer and the estimated state (the presence or absence and type of an APTT prolongation factor, and the like).

The database may include the results of determination of an APTT prolongation factor regarding the subject specimen performed in the facility. The determination results of the APTT prolongation factor may be transmitted to the database through the data transmission unit. In the present invention, through incorporation of the APTT prolongation factor of the subject specimen determined by the doctor into the database, the measurement data can be linked to clinical diagnosis, or the accuracy of the results of estimation by the computer can be improved.

## 1.2 APTT Prolongation Factor Estimation Algorithm

### 1.2.1 Template Matching

An algorithm for estimating the APTT prolongation factor of the subject specimen by the computer based on the measurement data on the subject specimen and the data stored in the database is not necessarily limited, and there is given, for example, template matching described below.

On the database, states related to an APTT prolongation factor in a blood specimen (for example, the presence or absence of an APTT prolongation factor, the presence or absence of various coagulation factor inhibitors, an inhibitor titer, a LA, coagulation factor deficiency, the type of a deficient coagulation factor, concentrations of various coagulation factors, and the like) and measurement data of blood coagulation measurement (data of a coagulation reaction curve or a primary differential curve thereof or a secondary differential curve thereof, or parameters related to a waveform or a weighted average point calculated from those curves, and the like) are linked to each other. As a result, the database provides templates for measurement data of blood coagulation measurement regarding specimens having various APTT prolongation factors. A specimen having the same APTT prolongation factor as that of the subject specimen can be selected from the database by inspecting which template the measurement data of the subject specimen is most approximate to by template matching. The APTT prolongation factor of the selected specimen may be estimated as the APTT prolongation factor of the subject specimen. Accordingly, as the number of specimens included in the database is increased, the accuracy of the results of the estimation of the APTT prolongation factor in the present invention may be improved.

In a more specific example of the template matching, regression analysis of corresponding parameters is performed between a parameter group related to a waveform or a weighted average point of the subject specimen (test parameter group) and a parameter group related to a waveform or a weighted average point of each specimen in the database (template parameter group) . A specimen in the database having the template parameter group that is most closely matched (for example, that has the highest correlation) with the test parameter group is selected. The APTT

prolongation factor of the selected specimen is estimated as the APTT prolongation factor of the subject specimen.

As the parameter group related to a waveform or a weighted average point to be used for the template matching, a parameter group comprising two or more types, preferably three or more types, more preferably five or more types of the parameters related to a waveform or a weighted average point shown in Table 3 is preferably used. When the parameter type is a parameter related to a weighted average point (for example, a weighted average time, a weighted average height, a peak width, a flatness, or a time rate), pieces of data (parameters) on preferably from 3 to 100 calculation target areas, more preferably from 5 to 20 calculation target areas (described later) are acquired for each parameter type. Then, a linear regression expression is obtained between the test parameter group and the template parameter group for each specimen in the database. For example, when the parameter group has a peak width vB and a flatness vAB related to a weighted average point of the primary differential curve in 5%, 10%, 20%, 30%, 40%, 50%, 60%, 70%, 80% and 90% of the calculation target area, vB5% of the test parameter group is plotted as X, and vB5% of the template parameter group is plotted as Y. Similarly, vB10% to vB90% and vAB5% to vAB90% are plotted. Then, a linear regression expression regarding the obtained plots is obtained. A specimen in the database that provides the template parameter group in which highly correlated regression line is obtained is selected. Preferably, a specimen in the database that provides a regression line having the highest correlation is selected.

Table 3

| Examples of parameters related to waveform or weighted average point included in parameter group to be used for template matching At time of primary differential curve F(t), secondary differential curve F' (t), and calculation target area value x% (t represents time) | | | |
|---|---|---|---|
| Parameters of F(t) | | Parameters of F'(t) | |
| Weighted average time vTx | X-coordinate of weighted average point | Weighted average time pTx | X-coordinate of weighted average point |
| Weighted average height vHx | Y-coordinate of weighted average point | Weighted average height pHx | Y-coordinate of weighted average point |
| Peak width vBx | Time width of F(t)≥x | Peak width pBx | Time width of F'(t)≥x |
| Weighted average peak width vWx | Time width of F(t)≥vHx | Weighted average peak width pWx | Time width of F' (t)≥pHx |
| Flatness vABx | vHx/vBx | Flatness pABx | pHx/pBx |
| Flatness vAWx | vHx/vWx | Flatness pAWx | pHx/pWx |
| Time rate vTBx | vTx/vBx | Time rate pTBx | pTx/pBx |
| Time rate vTWx | vTx/vWx | Time rate pTWx | pTx/pWx |
| Maximum velocity Vmax | Maximum value of F(t) | Maximum acceleration rate Amax | Maximum value of F'(t) |
| Maximum velocity time VmaxT | Time at which Vmax is achieved | Maximum acceleration rate time AmaxT | Time at which Amax is achieved |
| Peak area vAUC | Sum of height of F(t)≥x | Peak area pAUCx | Sum of height of F'(t)≥x |
| Coagulation time Tc | Reaction elapsed time until height c% is achieved when coagulation completion determination height is set to 100% in coagulation reaction curve | | |

Vmax(100%)
Weighted average
(vTx, vHx)
vWx
Corrected primary
differentiation
F(t)
vHx
vAUC
vBx
Calculation
target area
value x%
vTx
Tc
Time
vTex
vTsx

An example of a linear regression expression is shown in FIG. 1A. In FIG. 1A, there is shown a regression line of a parameter group between a subject specimen (Sample No. 67) and a specimen in the database (Template A) each having an FVIII activity of less than 0.2%. As the parameter group, there is used a parameter group consisting of a peak width vB, a weighted average time vT, a weighted average height vH, a flatness vAB, and a time rate vTB (50 parameters in total) regarding 10 calculation target areas (x=5%, 10%, 20%, 30%, 40%, 50%, 60%, 70%, 80% and 90%) of the primary differential curve. In FIG. 1B, there are shown primary differential curves (obtained by subjecting a coagulation reaction curve to zero adjustment and converting the resultant into relative values, followed by primary differentiation) of the subject specimen (Sample No. 67) and the reference (Template A) of FIG. 1A. The two curves have very similar shapes, indicating that the degree of approximation of blood coagulation characteristics is reflected in the correlation between the sample parameter groups.

In Table 4, there are shown examples of approximate index values (indices) (correlation coefficients) in which it is determined that the measurement data on the subject specimen and the measurement data on the specimen in the database are approximate to each other in the template matching based on the linear regression. As the approximate index values to be output as the results of the estimation, those from the highest rank to the preset rank N (N≥1) are displayed. The case "B" of Table 4 indicates the case of N=2.

Table 4

| Case | Estimation results |
|---|---|
| A | Severe hemophilia A (estimated FVIII:C <1%), Index: 0.98 |
| B | Mild hemophilia A (estimated FVIII:C 5% or more and less than 40%), Index: 0.95<br>Moderate hemophilia B (estimated FXII:C 1% or more and less than 5%), Index: 0.91 |
| C | Impossible to estimate |

1.2.2 Calculation of Parameters Related to Waveform or Weighted Average Point

A procedure for calculating parameters related to a waveform or a weighted average point from raw data on a coagulation reaction of a blood specimen obtained by an analyzer is described. The coagulation reaction curve is a curve

obtained by arranging the raw data on the coagulation reaction obtained by the analyzer in a chronological order. The coagulation reaction curve to be used for calculation of the parameters is preferably subjected to smoothing processing for removing noise and zero adjustment. In the zero adjustment, the data is corrected, for example, so that the value at the time of start of measurement is set to 0. Further, it is preferred that the data be corrected so that a maximum value of the coagulation reaction curve is set to a predetermined value (for example, 1 or 100) .

The above-mentioned corrected coagulation reaction curve is differentiated to create a primary differential curve and a secondary differential curve. The primary differential curve and the secondary differential curve each represent a change rate of the coagulation reaction curve.

A procedure for extracting parameters related to a waveform or a weighted average point through use of the primary differential curve as a target curve is described. Also in the secondary differential curve, parameters related to a waveform or a weighted average point can be extracted by a similar procedure. First, a calculation target area value x% of a target curve F(t) (t represents time) is determined. The calculation target area value x% is a value at which the F(t) reaches x% with respect to a maximum value (100%) thereof. An area in which F(t)≥x% is satisfied is referred to as "calculation target area." Two or more calculation target area values may be set with respect to one target curve. Next, weighted average points (vT, vH) of the F(t) for the calculation target area value (that is, a weighted average value in the calculation target area of the F(t)) are obtained. First, when the maximum value of the F(t) is Vmax, and the calculation target area value is "x" (% with respect to the Vmax), a time t [t1, ..., t2] (t1<t2) that satisfies "F(t)≥Vmax×x×0.01" is obtained, and a product sum value M is obtained by the following expression (1). The weighted average time vT and the weighted average height vH are calculated by the following expressions (2) and (3), respectively. The weighted average point is derived from the obtained vT and vH.

$$M = \sum_{i=t1}^{t2} (i \times F(i)) \qquad (1)$$

$$vT = \frac{M}{\sum_{i=t1}^{t2} F(i)} \qquad (2)$$

$$vH = \frac{M}{\sum_{i=t1}^{t2} i} \qquad (3)$$

The position of the weighted average point changes along with a change in calculation target area value "x". In order to identify the vT and the vH derived from different calculation target area values, the vT and the vH may be referred to as "vTx" and "vHx", respectively, in accordance with the calculation target area value "x" from which the vT and the vH are derived (see Table 3). For example, the vT and the vH of the calculation target area in which "x" is 5% are vT5% and vH5%.

Next, the peak width vB, the weighted average peak width vW, the flatness vAB and vAW, the time rates vTB and vTW, and the peak area vAUC are described. Those parameters are sometimes referred to as "vBx", "vWx", "vABx", "vAWx", "vTBx", "vTWx", and "vAUCx", respectively, in accordance with the calculation target area value "x" from which the parameters are derived, in order to identify the parameters derived from different calculation target area values (See Table 3). The "x" changes depending on the calculation target area value. For example, vBx, vWx, vABx, vAWx, vTBx, vTWx, and vAUCx in the calculation target area in which "x" is 5% are vBx5%, vWx5%, vAB5%, vAW5%, vTB5%, vTW5%, and vAUC5%, respectively.

When a maximum value and a minimum value of the time "t" that satisfies "F(t)≥x" described above are vTex and vTsx, respectively, a time length from the vTsx to the vTex, in which the F(t)≥x is satisfied, is defined as the peak width vBx of the F(t) regarding "x". In addition, the peak width satisfying "F(t)≥vH" is defined as a weighted average peak width vWx (see Table 3).

A flatness vABx [vABx=vHx/vBx] regarding "x" in the F(t), a flatness vAWx [vAWx=vHx/vWx] based on the weighted average peak width, a time rate vTBx [vTBx=vTx/vBx], and a time rate vTWx [vTWx=vTx/vWx] based on a weighted average peak width are calculated. The flatness may be "vABx=vBx/vHx" or "vAWx=vWx/vHx". Similarly, the time rate may be "vTBx=vBx/vTx" or "vTWx=vWx/vTx". In addition, those ratios may be multiplied by a constant K. Further, a peak area (area under a curve, vAUCx) in the calculation target area of the F(t) can be obtained (see Table 3).

The foregoing is the parameters related to a weighted average point of the F(t).

As parameters related to a waveform of the F(t), there are given the maximum value Vmax of the F(t) and an arrival time VmaxT, which is a time elapsed until the maximum value Vmax is reached. In addition, when a scattered light amount at the time when a change amount of the scattered light amount in the coagulation reaction curve satisfies predetermined conditions is 100%, the reaction elapsed time in which the scattered light amount corresponds to c% may be included, as a coagulation time Tc, in the parameters related to a waveform to be used in the present invention. The "c" may be any value, and for example, Tc is T50.

1.2.3 Others

The above-mentioned procedure for calculating parameters related to a waveform or a weighted average point, and the APTT prolongation factor estimation (diagnosis of hemophilia A, factor VIII quantification, and APTT cross-mixing test) method using those parameters may be used in the present invention as an APTT prolongation factor estimation algorithm to be used in the computer.

1.3 Machine Learning of APTT Prolongation Factor Estimation Algorithm

The APTT prolongation factor estimation system may comprise an algorithm learning unit configured to machine-learn an APTT prolongation factor estimation algorithm through use of the data on a blood coagulation reaction regarding a blood specimen and the data on an APTT prolongation factor which are stored in the database. The algorithm learning unit may be incorporated into the computer, or may be provided separately from the computer. For example, the algorithm learning unit is configured to construct a machine learning model for estimating an APTT prolongation factor of a blood specimen by machine leaning using the data on the blood coagulation reaction regarding the blood specimen as an explanatory variable and the data on the APTT prolongation factor regarding the blood specimen as an objective variable. As the data on the blood coagulation reaction to be used as the explanatory variable, there is given at least one selected from the group consisting of the above-mentioned measurement data (a coagulation reaction curve or a primary differential curve thereof or a secondary differential curve thereof, and parameters related to a waveform or a weighted average point of those curves; for example, a coagulation time, a weighted average time, a weighted average height, a peak width, a flatness, and a time rate, more specifically, Tc, vTx, vHx, vBx, vWx, vABx, vAWx, vTBx, vTWx, Vmax, VmaxT, vTsx, vTex, vAUCx, pTx, pHx, pBx, pWx, pABx, vAWx, pTBx, pTWx, Amax, AmaxT, and pAUCx (x is a freely-selected calculation target range value)). The machine learning model provides an APTT prolongation factor estimation algorithm that outputs an APTT prolongation factor (for example, coagulation factor deficiency, a LA, a coagulation factor inhibitor, and the like) of the subject specimen by inputting at least one selected from the group consisting of the above-mentioned measurement data regarding the subject specimen.

The algorithm constructed by the algorithm learning unit may be timely replaced by a related-art APTT prolongation factor estimation algorithm to be used in a computer (for example, the above-mentioned template matching using a regression line, or a machine learning model previously constructed by the algorithm learning unit). That is, the computer may estimate the APTT prolongation factor of the subject specimen by the APTT prolongation factor estimation algorithm constructed by machine learning in the algorithm learning unit.

Examples

Now, the present invention is described in detail by way of Examples, but the present invention is not limited to the following Examples.

(Example 1) Number of Templates (Specimen Data in Database) and Determination Accuracy in Template Matching

1) Subject specimen

The following 78 subject specimens were used: 70 cases of hemophilia A; 2 cases of acquired hemophilia A; 1 case of von Willebrand (vWD) Type 1; 3 cases of hemophilia B; and 2 cases of lupus anticoagulant (LA) positivity.

2) Template Specimen

143 Template specimens (Table 5: Ver. 1) or 158 template specimens (Table 5: Ver. 3) obtained by adding 15 additional specimens to the 143 template specimens were used.

Table 5

| Template specimen | Ver. 1 | Ver. 3 |
|---|---|---|
| ·George King FV Deficient having normal plasma added thereto in various concentrations | 10 | 10 |
| ·George King FX Deficient having normal plasma added thereto in various concentrations | 10 | 10 |
| ·George King FVIII Deficient having normal plasma added thereto in various concentrations | 10 | 10 |
| ·George King FIX Deficient having normal plasma added thereto in various concentrations | 10 | 10 |
| ·George King FXI Deficient having normal plasma added thereto in various concentrations | 10 | 10 |

(continued)

| Template specimen | Ver. 1 | Ver. 3 |
|---|---|---|
| ·George King FXII Deficient having normal plasma added thereto in various concentrations | 10 | 10 |
| ·George King PK Deficient having normal plasma added thereto in various concentrations | 10 | 10 |
| ·George King FVIII Deficient with inhibitor | 3 | 3 |
| ·George King FVIII Deficient having rFVIII Advate added thereto in various concentrations | 9 | 9 |
| ·Normal plasma (*1) having heparin added thereto | 9 | 9 |
| ·Barium sulfate adsorbed plasma (*2) | 2 | 2 |
| ·Specimen having anti-FVIII antibody added to normal plasma | 3 | 3 |
| ·LA positive specimen | 10 | 10 |
| ·Patient specimen | 37 | 52 |
| Total | 143 | 158 |

*1): CRYOcheck Pooled Normal Plasma (Precision BioLogic Incorporated)
*2): Barium sulfate adsorbed plasma was prepared by adding 5 (w/v)% to 20 (w/ v)% of barium sulfate to normal plasma, mixing the resultant at room temperat ure for from 10 minutes to 30 minutes, and then removing the barium sulfate.

3) Coagulation Reaction Measurement

A coagulation reaction of each of a subject specimen and a template specimen was measured. The coagulation reaction measurement was performed through use of an APTT measurement reagent Coagpia APTT-N (manufactured by Sekisui Medical Co., Ltd.) and a blood coagulation automatic analyzer CP3000 (manufactured by Sekisui Medical Co., Ltd.). 90-Degree laterally scattered light was measured to obtain a coagulation reaction curve. A primary differential curve was calculated from the obtained coagulation reaction curve.

4) Parameter Calculation

For subject specimens and template specimens, parameters related to a weighted average point of the primary differential curve were calculated. From the primary differential curve, calculation target areas were set in 10 stages of from 5% to 90% (calculation target area value "x" was set to 5%, 10%, 20%, 30%, 40%, 50%, 60%, 70%, 80%, and 90% with a maximum value Vmax of the primary differential curve being 100%). In each of the calculation target areas, the peak width vB, the weighted average time vT, the weighted average height vH, the flatness vAB=(vH/vB), and the time rate vTB=(vT/vB) were calculated.

Parameters for the 10 calculation target areas (x=5%, 10%, 20%, 30%, 40%, 50%, 60%, 70%, 80%, and 90%): vB [vB5%, vB10%, vB20%, vB30%, vB40%, vB50%, vB60%, vB70%, vB80%, and vB90%], vT [vT5%, vT10%, vT20%, vT30%, vT40%, vT50%, vT60%, vT70%, vT80%, and vT90%], vH [vH5%, vH10%, vH20%, vH30%, vH40%, vH50%, vH60%, vH70%, vH80%, and vH90%], vAB [vAB5%, vAB10%, vAB20%, vAB30%, vAB40%, vAB50%, vAB60%, vAB70%, vAB80%, and vAB90%], and vTB [vTB5%, vTB10%, vTB20%, vTB30%, vTB40%, vTB50%, vTB60%, vTB70%, vTB80%, and vTB90%] were calculated by the above-mentioned procedure. A parameter group consisting of those 50 parameters was created for each of the template specimens and subject specimens.

5) Template Matching

An APTT prolongation factor of each of the subject specimens was estimated by template matching. A linear regression expression for the parameter group was calculated between each of the subject specimens and each of all the template specimens. Template specimens having a regression expression with a slope of from 1.25 to 0.75 were extracted, and the template specimen having the largest correlation coefficient was selected from the extracted template specimens. Coagulation ability information of the template specimen was used as coagulation ability information of the subject specimen. Based on the analysis results, the coagulation ability information (FVIII concentration) of the subject specimen was classified into one of the following: FVIII<1% (FVIII activity of less than 1%), FVIII 1-5% (FVIII activity of 1% or more and less than 5%), FVIII 5-40% (FVIII activity of 5% or more and less than 40%), or "Other" (FVIII>40% (FVIII activity of 40% or more), or APTT prolongation caused by factors other than FVIII deficiency).

In FIG. 2A, there are shown primary differential curves of a subject specimen (Sample No. 57) and a template specimen (#027, FVIII activity of 0.6%, hemophilia A) having the highest correlation coefficient, and a regression line of a

parameter group between those specimens. In FIG. 2B, there are shown primary differential curves of Sample No. 57 and a template specimen (#134, FVIII activity of 0.25%, hemophilia A) having the second highest correlation coefficient, and a regression line of a parameter group between those specimens. Sample No. 57 was determined to be a specimen having coagulation ability information of the template #027 in accordance with this procedure and was classified as FVIII<1%. In actual measurement, the specimen was hemophilia A having FVIII activity of 0.3%.

The results of the template matching of the 78 subject specimens are as shown in Tables 6 and 7. In Table 6, there are shown an actually measured value of FVIII activity of each of the subject specimens and results of the estimation obtained by this procedure. In Table 7, there are shown matching rates between the results of the estimation and the actually measured values. As the number of templates was increased, the determination matching rate, sensitivity, specificity, and FVIII concentration classification matching rate were increased.

Table 6

| No. | Disease | APTT (sec) | FVIII (%) | | | No. | Disease | APTT (sec) | FVIII (%) | | |
|---|---|---|---|---|---|---|---|---|---|---|---|
| | | | Actual measurement | Estimation from Ver. 1 | Estimation from Ver. 3 | | | | Actual measurement | Estimation from Ver. 1 | Estimation from Ver. 3 |
| 1 | Hemophilia A | 40.1 | 62 | Other | Other | 41 | Hemophilia A | 69.3 | 1.3 | FVIII 1-5% | FVIII 1-5% |
| 2 | Hemophilia A | 40.5 | 13.7 | FVIII 5-40% | FVIII 5-40% | 42 | Hemophilia A | 72.8 | 1.2 | FVIII 5-40% | FVIII 1-5% |
| 3 | Hemophilia A | 40.8 | 20.1 | FVIII 5-40% | FVIII 5-40% | 43 | Hemophilia A | 73.3 | 3.1 | FVIII 1-5% | FVIII 1-5% |
| 4 | Hemophilia A | 41.7 | 10.4 | FVIII 5-40% | FVIII 5-40% | 44 | Hemophilia A | 73.4 | 1.1 | FVIII 1-5% | FVIII 1-5% |
| 5 | Hemophilia A | 42.4 | 12 | FVIII 5-40% | FVIII 5-40% | 45 | Hemophilia A | 74.1 | 0.9 | FVIII <1% | FVIII <1% |
| 6 | Hemophilia A | 44.3 | 15.7 | Other | Other | 46 | Hemophilia A | 74.1 | 1.8 | FVIII 1-5% | FVIII 1-5% |
| 7 | Hemophilia A | 44.4 | 14.8 | Other | FVIII 5-40% | 47 | Hemophilia A | 75.4 | 0.9 | FVIII 1-5% | FVIII 1-5% |
| 8 | Hemophilia A | 46.6 | 15 | FVIII 1-5% | FVIII 1-5% | 48 | Hemophilia A | 75.9 | 1.5 | FVIII 1-5% | FVIII 1-5% |
| 9 | Hemophilia A | 46.7 | 23.9 | Other | Other | 49 | Hemophilia A | 76.0 | 0.8 | FVIII 1-5% | FVIII 1-5% |
| 10 | Hemophilia A | 48.4 | 11.2 | FVIII 5-40% | FVIII 5-40% | 50 | Hemophilia A | 77.4 | 0.7 | FVIII 1-5% | FVIII 1-5% |
| 11 | Hemophilia A | 49.6 | 5.6 | FVIII 5-40% | FVIII 5-40% | 51 | Hemophilia A | 80.7 | 0.7 | FVIII 1-5% | FVIII 1-5% |
| 12 | Hemophilia A | 50.9 | 10.4 | FVIII 5-40% | FVIII 5-40% | 52 | Hemophilia A | 82.4 | 3.0 | FVIII 1-5% | FVIII 1-5% |
| 13 | Hemophilia A | 51.1 | 15 | Other | FVIII 5-40% | 53 | Hemophilia A | 87.2 | 0.3 | FVIII <1% | FVIII <1% |
| 14 | Hemophilia A | 51.5 | 9.6 | Other | FVIII 5-40% | 54 | Hemophilia A | 88.8 | 0.4 | FVIII <1% | FVIII <1% |
| 15 | Hemophilia A | 52.1 | 10.4 | FVIII 5-40% | FVIII 5-40% | 55 | Hemophilia A | 88.9 | 0.2 | FVIII <1% | FVIII <1% |
| 16 | Hemophilia A | 52.5 | 3.8 | FVIII 5-40% | FVIII 5-40% | 56 | Hemophilia A | 89.8 | 0.0 | FVIII <1% | FVIII <1% |
| 17 | Hemophilia A | 54.6 | 3.8 | FVIII 1-5% | FVIII 1-5% | 57 | Hemophilia A | 90.0 | 0.3 | FVIII <1% | FVIII <1% |
| 18 | Hemophilia A | 55.1 | 4.4 | FVIII 1-5% | FVIII 1-5% | 58 | Hemophilia A | 95.4 | 0.1 | FVIII <1% | FVIII <1% |
| 19 | Hemophilia A | 55.7 | 6.7 | FVIII 5-40% | FVIII 5-40% | 59 | Hemophilia A | 95.9 | 0.2 | FVIII <1% | FVIII <1% |
| 20 | Hemophilia A | 56 | 11.7 | Other | FVIII 1-5% | 60 | Hemophilia A | 96.7 | 0.2 | FVIII <1% | FVIII <1% |
| 21 | Hemophilia A | 56.2 | 3.4 | FVIII 5-40% | FVIII 1-5% | 61 | Hemophilia A | 97.9 | 0.2 | FVIII <1% | FVIII <1% |
| 22 | Hemophilia A | 56.4 | 4.8 | FVIII 1-5% | FVIII 1-5% | 62 | Hemophilia A | 98.8 | 0.4 | FVIII <1% | FVIII <1% |
| 23 | Hemophilia A | 57.7 | 4.6 | FVIII 1-5% | FVIII 1-5% | 63 | Hemophilia A | 99.1 | 0.2 | FVIII <1% | FVIII <1% |
| 24 | Hemophilia A | 57.8 | 5.4 | FVIII 1-5% | FVIII 1-5% | 64 | Hemophilia A | 103.2 | 0.2 | FVIII <1% | FVIII <1% |
| 25 | Hemophilia A | 57.9 | 2.9 | FVIII 1-5% | FVIII 1-5% | 65 | Hemophilia A | 103.4 | 0.3 | FVIII <1% | FVIII <1% |
| 26 | Hemophilia A | 57.9 | 8.8 | FVIII 5-40% | FVIII 5-40% | 66 | Hemophilia A | 110.2 | 0.1 | FVIII <1% | FVIII <1% |
| 27 | Hemophilia | 59.5 | 4.6 | FVIII <1% | FVIII <1% | 67 | Hemophilia | 115.8 | 0.2 | FVIII <1% | FVIII <1% |

| | | | | | | | | | | | |
|---|---|---|---|---|---|---|---|---|---|---|---|
| | A | | | | | | A | | | | |
| 28 | Hemophilia A | 59.8 | 4.7 | FVIII 5-40% | FVIII 5-40% | 68 | Hemophilia A | 122.2 | 0.1 | FVIII <1% | FVIII <1% |
| 29 | Hemophilia A | 61 | 4.6 | FVIII 1-5% | FVIII 1-5% | 69 | Hemophilia A | 141.6 | 0.2 | FVIII <1% | FVIII <1% |
| 30 | Hemophilia A | 61.4 | 12.2 | FVIII 1-5% | FVIII 1-5% | 70 | Hemophilia A | 144.1 | 0.3 | FVIII <1% | FVIII <1% |
| 31 | Hemophilia A | 61.7 | 4.4 | FVIII 1-5% | FVIII 1-5% | 71 | Acquired hemophilia | 68.9 | 7.8 | FVIII 1-5% | FVIII 1-5% |
| 32 | Hemophilia A | 62.1 | 3.4 | FVIII 5-40% | FVIII 5-40% | 72 | Acquired hemophilia | 123.3 | 0.2 | FVIII <1% | FVIII <1% |
| 33 | Hemophilia A | 63.5 | 3.1 | FVIII 5-40% | FVIII 5-40% | 73 | vWD type 1 | 40.6 | 16.5 | FVIII 5-40% | FVIII 5-40% |
| 34 | Hemophilia A | 65.4 | 1.1 | FVIII 1-5% | FVIII 1-5% | 74 | Hemophilia B | 49.2 | | Other | Other |
| 35 | Hemophilia A | 65.8 | 3.5 | FVIII 1-5% | FVIII 1-5% | 75 | Hemophilia B | 54.3 | | Other | Other |
| 36 | Hemophilia A | 66.2 | 1.5 | FVIII 1-5% | FVIII 1-5% | 76 | Hemophilia B | 64.1 | | Other | Other |
| 37 | Hemophilia A | 66.6 | 2.7 | FVIII 1-5% | FVIII 1-5% | 77 | LA | 41.1 | | FVIII 5-40% | Other |
| 38 | Hemophilia A | 67.4 | 3.5 | FVIII 1-5% | FVIII 1-5% | 78 | LA | 92.6 | | FVIII <1% | FVIII <1% |
| 39 | Hemophilia A | 68.8 | 0.9 | FVIII <1% | FVIII <1% | | | | | | |
| 40 | Hemophilia A | 69 | 1.4 | FVIII 5-40% | FVIII 1-5% | | | | | | |

Enclosed portion: Determination results of hemophilia A are incorrect
Underline: Estimation results of FVIII concentration are incorrect

Table 7

| Template specimen (Number of templates) | Ver. 1 (N=143) | Ver. 3 (N=158) |
|---|---|---|
| Determination matching rate of hemophilia A | 84.6% | 89.7% |
| Sensitivity | 91.7% | 97.2% |
| Specificity | 66.7% | 83.3% |
| FVIII concentration classification matching rate | 69.2% | 78.2% |

(Example 2) Correction of Difference between Reagent Lots using Controlled specimen

1) Specimen

As a normal specimen, citrated plasma obtained from a healthy person was used.

As a hemophilia specimen, citrated plasma of a hemophilia A patient was used.

2) Measurement of Coagulation Reaction of Specimen

For APTT measurement, a blood coagulation automatic analyzer CP3000 (manufactured by Sekisui Medical Co., Ltd.) was used. After 50 μL of a measurement sample was discharged into a cuvette (reaction vessel), heating was performed at 37°C for 45 seconds. Then, 50 μL of an APTT reagent heated to about 37°C was discharged to the cuvette, and after an elapse of 171 seconds, 50 μL of 25 mM calcium chloride solution was discharged to the cuvette to start a coagulation reaction. In this case, two lots of the APTT reagent (Lot A and Lot B) were used. The coagulation reaction was performed under a state in which the cuvette was kept at about 37°C. The coagulation reaction was detected by radiating light from an LED having a wavelength of 660 nm as a light source and measuring the amount of scattered light scattered laterally at an angle of 90 degrees at intervals of 0.1 second. The photometric time was set to 360 seconds.

3) Method of Analyzing Photometric Data

APTT was measured, and photometric raw data was obtained as coagulation reaction data by APTT measurement. After the photometric raw data was subjected to smoothing processing including noise removal, zero adjustment was performed so that the amount of scattered light at the time of the start of photometry reached 0, and thus a coagulation

reaction curve was obtained. Subsequently, correction processing was performed so that the maximum height of the coagulation reaction curve reached 100 to obtain a corrected coagulation reaction curve. The corrected coagulation reaction curve was defined as a zero-order waveform, and the zero-order waveform was subjected to primary differentiation to obtain a first-order waveform reflecting a coagulation rate. Weighted average method parameters were obtained from the first-order waveform.

4) APTT Difference Correction between Lots

The correlation of the weighted average method waveform parameters obtained from the measurement of controlled specimens of the Lot A and the Lot B of the APTT reagent was analyzed, and the weighted average method parameters of the Lot B were approximated to the values of the Lot A through use of the obtained regression expression (y=0.965x+0.13).

5) Confirmation of Effects of APTT Difference Correction between Lots

The results of template matching using weighted average waveform parameters of the Lot A, the Lot B, and the corrected Lot B were compared to each other through use of eight patient specimens. As shown in Table 8, there was a discrepancy in determination when the difference between the lots was not corrected, but the corrected Lot B and the Lot A were matched with each other. The determination result "Other" of Table 8 indicates the case in which a specimen was determined to have prolonged APTT due to a factor other than hemophilia A.

Table 8

Determination results of template matching

| | Lot A | | Lot B | Corrected Lot B |
|---|---|---|---|---|
| 1 | Other | | Other | Other |
| 2 | FVIII | 0.2-1% | FVIII 0.2-1% | FVIII 0.2-1% |
| 3 | FVIII | <0.2% | FVIII <0.2% | FVIII <0.2% |
| 4 | FVIII | 1-5% | FVIII 1-5% | FVIII 1-5% |
| 5 | Other | | Other | Other |
| 6 | Other | | Other | Other |
| 7 | FVIII | 0.2-1% | FVIII 1-5% | FVIII 0.2-1% |
| 8 | FVIII | 5-40% | FVIII 5-40% | FVIII 5-40% |

## Claims

**1.** An APTT prolongation factor estimation system, comprising:

more than one facilities each comprising an analyzer which measures a blood coagulation reaction of a subject blood specimen;
a database which stores data on a blood coagulation reaction and APTT prolongation factor of blood specimens; and
a computer which estimates an APTT prolongation factor of the subject blood specimen based on data on the blood coagulation reaction from the analyzer and the data stored in the database,
wherein the more than one facilities each further comprise:

a data transmission unit which transmits, to the computer, the data on the blood coagulation reaction of the subject blood specimen obtained by the analyzer; and
a data reception unit which receives a result the estimation of the APTT prolongation factor, which is obtained by the computer, regarding the subject blood specimen measured by each of the analyzers,

wherein the data on the blood coagulation reaction of the subject blood specimen transmitted to the computer and the result of the estimation of the APTT prolongation factor regarding the subject blood specimen obtained by the computer are stored in the database.

**2.** The system according to claim 1, wherein the more than one facilities each further comprise:

an analyzer control unit which controls the analyzer; and/or
a data display unit which displays data received by the data reception unit.

**3.** The system according to claim 1 or 2, further comprising an algorithm learning unit which machine-learns an APTT prolongation factor estimation algorithm through use of the data on the blood coagulation reaction and the APTT prolongation factor of the blood specimens which are stored in the database.

**4.** The system according to any one of claims 1 to 3, wherein the data on the blood coagulation reaction comprises one or more parameters related to a waveform or a weighted average point regarding a coagulation reaction curve, or a primary differential curve of the coagulation reaction curve or a secondary differential curve of the coagulation reaction curve.

**5.** The system according to claim 4, wherein the parameters related to the waveform are selected from a coagulation time, a maximum peak height of the primary differential curve or the secondary differential curve, and a time elapsed until the maximum peak height is reached, and the parameters related to the weighted average point are selected from a weighted average time, a weighted average height, a peak width, a flatness, a time rate, and a peak area regarding a calculation target area of the primary differential curve or the secondary differential curve.

**6.** The system according to claim 5, wherein the parameters related to the weighted average point comprise one or more parameters selected from the weighted average time, the weighted average height, the peak width, the flatness, the time rate, and the peak area, which are acquired for each of two or more calculation target areas of the primary differential curve.

**7.** The system according to claim 6, wherein, when the primary differential curve is represented by F(t) (t represents time) and a time when the F(t) is a predetermined value "x" is represented by t1 and t2 (t1<t2), the calculation target area is a region in which F(t)≥x is satisfied, and the weighted average time and the weighted average height are expressed as vT and vH represented by the following expressions, respectively:

$$vT = \frac{M}{\sum_{t=t1}^{t2} F(t)} \quad (2)$$

$$vH = \frac{M}{\sum_{t=t1}^{t2} t} \quad (3)$$

where

$$M = \sum_{t=t1}^{t2} (t \times F(t)) \quad (1)$$

wherein the peak width is expressed as vB, which is a length of time satisfying F(t)≥x from the t1 to the t2,
wherein the flatness is expressed as vAB, which is a ratio between the vH and the vB,
wherein the time rate is expressed as vTB, which is a ratio between the vT and the vB, and
wherein the peak area is expressed as f, which is an area under a curve.

**8.** The system according to claim 7, wherein the predetermined value "x" is from 5% to 90% of a maximum value of the F(t).

**9.** The system according to any one of claims 4 to 8, wherein the estimation of the APTT prolongation factor of the subject blood specimen is performed by selection, from the blood specimens of which data are stored in the database, of a specimen in which the one or more parameters related to the waveform or the weighted average point are approximate to parameters of the subject blood specimen, and estimation of an APTT prolongation factor of the selected specimen as an APTT prolongation factor of the subject blood specimen.

**10.** The system according to claim 9, wherein the selection of the specimen is performed through use of regression analysis.

**11.** The system according to claim 9, wherein the selection of the specimen is performed through use of an APTT

prolongation factor estimation algorithm obtained by machine learning.

**12.** The system according to any one of claims 1 to 11, wherein the data on the APTT prolongation factor is information on coagulation factor deficiency, lupus anticoagulant positivity or presence or absence of a coagulation factor inhibitor, or a coagulation factor concentration.

**13.** The system according to claim 12, wherein the estimation of the APTT prolongation factor of the subject blood specimen comprises estimation of the coagulation factor deficiency, the lupus anticoagulant positivity, or the presence or absence of a coagulation factor inhibitor as the APTT prolongation factor of the subject blood specimen.

**14.** The system according to claim 12, wherein the estimation of the APTT prolongation factor of the subject blood specimen comprises estimation of the coagulation factor concentration of the subject blood specimen.

**15.** The system according to any one of claims 1 to 14,

wherein the data transmission unit transmits data on a blood coagulation reaction of a controlled specimen to the computer,
wherein the computer compares the data on the blood coagulation reaction of the controlled specimen to data on a blood coagulation reaction of a past controlled specimen measured through use of a reagent of a different lot,
wherein data measured by the analyzer is calibrated based on results of the comparison, and
wherein the controlled specimen is a blood specimen having a known APTT prolongation factor, and the past controlled specimen is the same specimen as the controlled specimen or a specimen having the same APTT prolongation factor as the APTT prolongation factor of the controlled specimen.

**16.** An APTT prolongation factor estimation method, comprising:

measuring a blood coagulation reaction of a subject blood specimen in each of more than one facilities;
transmitting, from each of the one or more facilities, data on the blood coagulation reaction of the subject blood specimen acquired in the each of the more than one facilities to a computer;
estimating, by the computer, an APTT prolongation factor of the subject blood specimen based on the data on the blood coagulation reaction of the subject blood specimen, and data on a blood coagulation reaction and APTT prolongation factor of blood specimens which are stored in a database; and
transmitting a result of the estimation of the APTT prolongation factor of the subject blood specimen obtained by the computer to each of the more than one facilities in which the blood coagulation reaction of the subject blood specimen has been measured.

**Patentansprüche**

**1.** System zum Abschätzen eines APTT-Verlängerungsfaktors, umfassend:

mehr als eine Einrichtung, die jeweils ein Analysegerät umfasst, der eine Blutgerinnungsreaktion einer Blutprobe einer Testperson misst;
eine Datenbank, die Daten zu einer Blutgerinnungsreaktion und einem APTT-Verlängerungsfaktor von Blutproben speichert; und
einen Computer, der einen APTT-Verlängerungsfaktor der Blutprobe der Testperson auf der Grundlage von Daten der Blutgerinnungsreaktion aus dem Analysegerät und den in der Datenbank gespeicherten Daten abschätzt,
wobei die mehr als eine Einrichtung jeweils ferner umfassen:

eine Datenübertragungseinheit, die vom Analysegerät ermittelte Daten zur Blutgerinnungsreaktion der Blutprobe der Testperson an den Computer überträgt; und
eine Datenempfangseinheit, die ein vom Computer ermitteltes Ergebnis der Abschätzung des APTT-Verlängerungsfaktors bezüglich der von jedem der Analysegeräte gemessenen Blutprobe einer Testperson empfängt,

wobei die Daten zur Blutgerinnungsreaktion der Blutprobe einer Testperson, die an den Computer übertragen wurden, sowie das vom Computer ermittelte Ergebnis der Abschätzung des APTT-Verlängerungsfaktors

bezüglich der Blutprobe einer Testperson in der Datenbank gespeichert werden.

**2.** Das System nach Anspruch 1, wobei die mehr als eine Einrichtung jeweils ferner umfasst:

eine Analysegerät-Steuereinheit, die das Analysegerät steuert; und/oder
eine Datenanzeigeeinheit, die von der Datenempfangseinheit empfangenen Daten anzeigt.

**3.** Das System nach Anspruch 1 oder 2, das ferner eine Algorithmus-Lerneinheit umfasst, die einen Algorithmus zur Abschätzung des APTT-Verlängerungsfaktors mittels maschinellen Lernens unter Verwendung der in der Datenbank gespeicherten Daten zur Blutgerinnungsreaktion und zum APTT-Verlängerungsfaktor der Blutproben trainiert.

**4.** Das System nach einem der Ansprüche 1 bis 3, wobei die Daten zur Blutgerinnungsreaktion einen oder mehrere Parameter umfassen, die sich auf eine Wellenform oder einen gewichteten Durchschnittspunkt in Bezug auf eine Gerinnungsreaktionskurve oder eine primäre Differentialkurve der Gerinnungsreaktionskurve oder eine sekundäre Differentialkurve der Gerinnungsreaktionskurve beziehen.

**5.** Das System nach Anspruch 4, wobei die auf die Wellenform bezogenen Parameter aus einer Gerinnungszeit, einer maximalen Spitzenhöhe der primären Differentialkurve oder der sekundären Differentialkurve und einer bis zum Erreichen der maximalen Spitzenhöhe verstrichenen Zeit ausgewählt sind, und die auf den gewichteten Durchschnittspunkt bezogenen Parameter aus einer gewichteten Durchschnittszeit, einer gewichteten Durchschnittshöhe, einer Peakbreite, einer Flachheit, einer Zeitrate und einer Peakfläche in Bezug auf einen Berechnungszielbereich der primären Differentialkurve oder der sekundären Differentialkurve ausgewählt werden.

**6.** Das System nach Anspruch 5, wobei die Parameter, die sich auf den gewichteten Durchschnittspunkt beziehen, einen oder mehrere Parameter umfassen, die aus der gewichteten Durchschnittszeit, der gewichteten Durchschnittshöhe, der Peakbreite, der Flachheit, der Zeitrate und der Peakfläche ausgewählt werden, die für jeden von zwei oder mehr Berechnungszielbereichen der primären Differentialkurve erfasst werden.

**7.** Das System gemäß Anspruch 6,

wobei, wenn die primäre Differentialkurve durch F(t) dargestellt wird (t steht für die Zeit) und ein Zeitpunkt, zu dem F(t) einen vorbestimmten Wert "x" annimmt, durch t1 und t2 (t1 < t2) dargestellt wird, der Berechnungszielbereich ein Bereich ist, in dem F(t) ≥ x erfüllt ist, und die gewichtete Durchschnittszeit sowie die gewichtete Durchschnittshöhe als vT bzw. vH ausgedrückt werden, die durch die folgenden Ausdrücke dargestellt werden:

$$vT = \frac{M}{\sum_{t=t1}^{t2} F(t)} \qquad (2)$$

$$vH = \frac{M}{\sum_{t=t1}^{t2} t} \qquad (3)$$

wobei

$$M = \sum_{t=t1}^{t2} (t \times F(t)) \qquad (1)$$

wobei die Peakbreite als vB ausgedrückt wird, was einer Zeitspanne entspricht, in der F(t) ≥ x von $t_1$ bis $t_2$ gilt,
wobei die Flachheit als vAB ausgedrückt wird, was ein Verhältnis zwischen vH und vB ist,
wobei die Zeitrate als vTB ausgedrückt wird, was ein Verhältnis zwischen vT und vB ist, und
wobei die Peakfläche als f ausgedrückt wird, was die Fläche unter einer Kurve ist.

**8.** Das System nach Anspruch 7, wobei der vorgegebene Wert "x" zwischen 5 % und 90 % eines Maximalwerts von F(t) liegt.

**9.** System nach einem der Ansprüche 4 bis 8, wobei die Abschätzung des APTT-Verlängerungsfaktors der Blutprobe einer Testperson dadurch erfolgt, dass aus den Blutproben, deren Daten in der Datenbank gespeichert sind, eine

Probe ausgewählt wird, bei der der eine oder die mehreren Parameter, die sich auf die Wellenform oder den gewichteten Durchschnittspunkt beziehen, den Parametern der Blutprobe einer Testperson nahekommen, und durch Schätzung eines APTT-Verlängerungsfaktors der ausgewählten Probe als APTT-Verlängerungsfaktor der Blutprobe einer Testperson.

**10.** Das System nach Anspruch 9, wobei die Auswahl der Probe unter Verwendung einer Regressionsanalyse erfolgt.

**11.** Das System nach Anspruch 9, wobei die Auswahl der Probe unter Verwendung eines durch maschinelles Lernen gewonnenen Algorithmus zur Schätzung des APTT-Verlängerungsfaktors erfolgt.

**12.** Das System nach einem der Ansprüche 1 bis 11, wobei die Daten zum APTT-Verlängerungsfaktor Informationen über einen Gerinnungsfaktormangel, die Positivität auf Lupus-Antikoagulans oder das Vorhandensein bzw. Fehlen eines Gerinnungsfaktor-Inhibitors oder eine Gerinnungsfaktorkonzentration sind.

**13.** Das System nach Anspruch 12, wobei die Abschätzung des APTT-Verlängerungsfaktors der Blutprobe einer Testperson die Schätzung des Gerinnungsfaktormangels, der Lupus-Antikoagulans-Positivität oder des Vorhandenseins bzw. Fehlens eines Gerinnungsfaktor-Inhibitors als APTT-Verlängerungsfaktor der Blutprobe einer Testperson umfasst.

**14.** Das System gemäß Anspruch 12, wobei die Bestimmung des APTT-Verlängerungsfaktors der Blutprobe einer Testperson die Bestimmung der Gerinnungsfaktorkonzentration der Blutprobe der Testperson umfasst.

**15.** Das System nach einem der Ansprüche 1 bis 14,

wobei die Datenübertragungseinheit Daten über eine Blutgerinnungsreaktion einer Kontrollprobe an den Computer überträgt,
wobei der Computer die Daten über die Blutgerinnungsreaktion der Kontrollprobe mit Daten über eine Blutgerinnungsreaktion einer früheren untersuchten Kontrollprobe vergleicht, die unter Verwendung eines Reagenzes einer anderen Charge gemessen wurde,
wobei die vom Analysegerät gemessenen Daten auf der Grundlage der Ergebnisse des Vergleichs kalibriert werden, und
wobei die Kontrollprobe eine Blutprobe mit einem bekannten APTT-Verlängerungsfaktor ist und die frühere Kontrollprobe dieselbe Probe wie die Kontrollprobe oder eine Probe mit demselben APTT-Verlängerungsfaktor wie der APTT-Verlängerungsfaktor der Kontrollprobe ist.

**16.** Verfahren zur Abschätzung eines APTT-Verlängerungsfaktors, umfassend:

Messen einer Blutgerinnungsreaktion einer Blutprobe einer Testperson in jeder von mehr als einer Einrichtung;
Übertragen von Daten zur Blutgerinnungsreaktion der Blutprobe der Testperson, die in jeder der einen oder mehreren Einrichtungen erfasst wurden, von jeder der mehr als einen Einrichtungen an einen Computer;
Abschätzen eines APTT-Verlängerungsfaktors der Blutprobe einer Testperson durch den Computer auf der Grundlage der Daten zur Blutgerinnungsreaktion der Blutprobe einer Testperson sowie von Daten zur Blutgerinnungsreaktion und zum APTT-Verlängerungsfaktor von Blutproben, die in einer Datenbank gespeichert sind; und
Übertragen eines vom Computer ermittelten Ergebnisses der Abschätzung des APTT-Verlängerungsfaktors der Blutprobe einer Testperson an jede der mehr als eine Einrichtung, in der die Blutgerinnungsreaktion der Blutprobe einer Testperson gemessen wurde.

## Revendications

**1.** Système d’estimation de facteur de prolongation d’APTT, comprenant :

plus d’une installation comprenant chacune un analyseur qui mesure une réaction de coagulation sanguine d’un échantillon de sang du sujet ;
une base de données qui stocke des données sur une réaction de coagulation sanguine et un facteur de prolongation d’APTT d’échantillons de sang ; et
un ordinateur qui estime un facteur de prolongation d’APTT de l’échantillon de sang du sujet sur la base de

données sur la réaction de coagulation sanguine provenant de l'analyseur et des données stockées dans la base de données,
dans lequel les plus d'une installations comprennent en outre chacune :

une unité de transmission de données qui transmet, à l'ordinateur, les données sur la réaction de coagulation sanguine de l'échantillon de sang du sujet obtenues par l'analyseur ; et
une unité de réception de données qui reçoit un résultat de l'estimation du facteur de prolongation d'APTT, qui est obtenu par l'ordinateur, concernant l'échantillon de sang du sujet mesuré par chacun des analyseurs, dans lequel les données sur la réaction de coagulation sanguine de l'échantillon de sang du sujet transmises à l'ordinateur et le résultat de l'estimation du facteur de prolongation d'APTT concernant l'échantillon de sang du sujet obtenu par l'ordinateur sont stockées dans la base de données.

**2.** Système selon la revendication 1, dans lequel les plus d'une installations comprennent en outre chacune :

une unité de commande d'analyseur qui commande l'analyseur ; et/ou
une unité d'affichage de données qui affiche les données reçues par l'unité de réception de données.

**3.** Système selon la revendication 1 ou 2, comprenant en outre une unité d'apprentissage d'algorithme qui apprend automatiquement un algorithme d'estimation du facteur de prolongation d'APTT par l'utilisation des données sur la réaction de coagulation sanguine et le facteur de prolongation d'APTT des échantillons de sang qui sont stockés dans la base de données.

**4.** Système selon l'une des revendications 1 à 3, dans lequel les données sur la réaction de coagulation sanguine comprennent un ou plusieurs paramètres liés à une forme d'onde ou un point moyen pondéré concernant une courbe de réaction de coagulation, ou une courbe différentielle primaire de la courbe de réaction de coagulation ou une courbe différentielle secondaire de la courbe de réaction de coagulation.

**5.** Système selon la revendication 4, dans lequel les paramètres liés à la forme d'onde sont sélectionnés parmi un temps de coagulation, une hauteur de pic maximale de la courbe différentielle primaire ou de la courbe différentielle secondaire, et un temps écoulé jusqu'à ce que la hauteur de pic maximale soit atteinte, et les paramètres liés au point moyen pondéré sont sélectionnés parmi un temps moyen pondéré, une hauteur moyenne pondérée, une largeur de pic, une planéité, un taux de temps, et une zone de pic concernant une zone cible de calcul de la courbe différentielle primaire ou de la courbe différentielle secondaire.

**6.** Système selon la revendication 5, dans lequel les paramètres liés au point moyen pondéré comprennent un ou plusieurs paramètres sélectionnés parmi le temps moyen pondéré, la hauteur moyenne pondérée, la largeur de pic, la planéité, le taux de temps et la zone de pic, qui sont acquis pour chacune de deux zones cibles de calcul ou plus de la courbe différentielle primaire.

**7.** Système selon la revendication 6,

dans lequel, lorsque la courbe différentielle primaire est représentée par F(t) (t représente le temps) et qu'un moment où la F(t) est une valeur prédéterminée « x » est représenté par t1 et t2 (t1<t2), la zone cible de calcul est une région dans laquelle F(t) ≥ x est satisfaite, et le temps moyen pondéré et la hauteur moyenne pondérée sont exprimés en vT et vH représentés par les expressions suivantes, respectivement :

$$vT = \frac{M}{\sum_{t=t1}^{t2} F(t)} \quad (2)$$

$$vH = \frac{M}{\sum_{t=t1}^{t2} t} \quad (3)$$

où

$$M = \sum_{t=t1}^{t2} (t \times F(t)) \quad (1)$$

dans lequel la largeur de pic est exprimée en vB, qui est une durée satisfaisant F(t) ≥x du t1 au t2,
dans lequel la planéité est exprimée en vAB, qui est un rapport entre le vH et la vB,
dans lequel le taux de temps est exprimé en vTB, qui est un rapport entre le vT et la vB, et
dans lequel la zone de pic est exprimée en f, qui est une zone sous une courbe.

**8.** Système selon la revendication 7, dans lequel la valeur prédéterminée « x » est de 5 % à 90 % d'une valeur maximale de la F(t).

**9.** Système selon l'une des revendications 4 à 8, dans lequel l'estimation du facteur de prolongation d'APTT de l'échantillon de sang du sujet est effectuée par sélection, parmi les échantillons de sang dont les données sont stockées dans la base de données, d'un échantillon dans lequel les un ou plusieurs paramètres liés à la forme d'onde ou au point moyen pondéré sont approximatifs de paramètres de l'échantillon de sang du sujet, et l'estimation d'un facteur de prolongation d'APTT de l'échantillon sélectionné en tant que facteur de prolongation d'APTT de l'échantillon de sang du sujet.

**10.** Système selon la revendication 9, dans lequel la sélection de l'échantillon est effectuée par l'utilisation d'une analyse de régression.

**11.** Système selon la revendication 9, dans lequel la sélection de l'échantillon est effectuée par l'utilisation d'un algorithme d'estimation du facteur de prolongation d'APTT obtenu par apprentissage automatique.

**12.** Système selon l'une des revendications 1 à 11, dans lequel les données sur le facteur de prolongation d'APTT sont des informations sur un déficit en facteur de coagulation, une positivité aux anticoagulants lupiques ou la présence ou l'absence d'un inhibiteur de facteur de coagulation, ou une concentration de facteur de coagulation.

**13.** Système selon la revendication 12, dans lequel l'estimation du facteur de prolongation d'APTT de l'échantillon de sang du sujet comprend l'estimation de la carence en facteur de coagulation, de la positivité aux anticoagulants lupiques, ou de la présence ou de l'absence d'un inhibiteur du facteur de coagulation en tant que facteur de prolongation d'APTT de l'échantillon de sang du sujet.

**14.** Système selon la revendication 12, dans lequel l'estimation du facteur de prolongation d'APTT de l'échantillon de sang du sujet comprend l'estimation de la concentration de facteur de coagulation de l'échantillon de sang du sujet.

**15.** Système selon l'une des revendications 1 à 14,

dans lequel l'unité de transmission de données transmet des données sur une réaction de coagulation sanguine d'un échantillon contrôlé à l'ordinateur,
dans lequel l'ordinateur compare les données sur la réaction de coagulation sanguine de l'échantillon contrôlé à des données sur une réaction de coagulation sanguine d'un ancien échantillon contrôlé mesurée par l'utilisation d'un réactif d'un lot différent,
dans lequel les données mesurées par l'analyseur sont étalonnées sur la base des résultats de la comparaison, et
dans lequel l'échantillon contrôlé est un échantillon de sang ayant un facteur de prolongation d'APTT connu, et l'ancien échantillon contrôlé est le même échantillon que l'échantillon contrôlé ou un échantillon ayant le même facteur de prolongation d'APTT que le facteur de prolongation d'APTT de l'échantillon contrôlé.

**16.** Procédé d'estimation de facteur de prolongation d'APTT, comprenant :

la mesure d'une réaction de coagulation sanguine d'un échantillon de sang du sujet dans chacune de plus d'une installation ;
la transmission, à partir de chacune des une ou plusieurs installations, de données sur la réaction de coagulation sanguine de l'échantillon de sang du sujet acquises dans chacune des plus d'une installations à un ordinateur ;
l'estimation, par l'ordinateur, d'un facteur de prolongation d'APTT de l'échantillon de sang du sujet sur la base des données sur la réaction de coagulation sanguine de l'échantillon de sang du sujet, et des données sur une réaction de coagulation sanguine et un facteur de prolongation d'APTT d'échantillons de sang qui sont stockés dans une base de données ; et
la transmission d'un résultat de l'estimation du facteur de prolongation d'APTT de l'échantillon de sang du sujet obtenu par l'ordinateur à chacune des plus d'une installations dans lesquelles la réaction de coagulation sanguine de l'échantillon de sang du sujet a été mesurée.

FIG. 1A
y = 1.1557x + 0.4902
R² = 0.9985
Parameters of Template
250
200
150
100
50
0
0
50
100
150
200
250
Parameters of Sample
FIG. 1B
PRIMARY DIFFERENTIAL VALUE
0.10
0.08
0.06
0.04
0.02
0.00
Sample no. 67
Template A
0
100
200
300
400
Time (sec)

FIG. 2A
FIG. 2B
PRIMARY DIFFERENTIAL VALUE
Time (sec)
Template #027
Sample no.57
Template #134
Sample no.57
y = 0.9305x + 0.1174
R² = 0.9989
y = 1.0291x + 0.6109
R² = 0.9831
Parameters of Template
Parameters of Sample

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6898532 B1
- JP 2016194426 A
- JP 2016118442 A
- JP 2017106925 A
- JP 2018017619 A
- JP 2018517150 A
- US 6524861 B1
- JP 2017187473 A
- JP 2016508610 A
- JP 2019086658 A
- JP 2019084727 A
- JP 2019016474 A


### Non-patent literature cited in the description


- *British Journal of Haematology*, 1997, vol. 98, 68-73
- *The Japanese Journal of Clinical Hematology*, 2017, vol. 58 (9), 1754
- *Japanese Journal of Thrombosis and Hemostasis*, 2018, vol. 29 (2), 184
- *Japanese Journal of Thrombosis and Hemostasis*, 2018, vol. 29 (4), 413-420